(19) Europäisches Patentamt
European Patent Office
Office européen des brevets

(11) Publication number : **0 686 628 A2**

# (12) EUROPEAN PATENT APPLICATION

(21) Application number : **95301909.8**

(22) Date of filing : **22.03.95**

(51) Int. Cl.$^6$ : **C07C 405/00,** // A61K31/557

(30) Priority : **22.03.94 JP 75381/94**

(43) Date of publication of application :
**13.12.95 Bulletin 95/50**

(84) Designated Contracting States :
**AT BE CH DE DK ES FR GB GR IE IT LI LU MC NL PT SE**

(71) Applicant : **ONO PHARMACEUTICAL CO., LTD.**
**1-5, Doshomachi 2-chome**
**Chuo-ku Osaka 541 (JP)**

(72) Inventor : **Miyazaki, Tohru, c/o Minase Res. Inst.**
**Ono Pharmaceutical Co., Ltd,**
**1-1, Sakurai 3-chome**
**Shimamoto-cho, Mishima-gun, Osaka 618 (JP)**
Inventor : **Kawamura, Masanori, c/o Minase Res. Inst.**
**Ono Pharmaceutical Co., Ltd,**
**1-1, Sakurai 3-chome**
**Shimamoto-cho, Mishima-gun, Osaka 618 (JP)**
Inventor : **Shirasawa, Eiichi**
**1-1, Ohe Kitafukunishi-cho 4-chome,**
**Nishikyo-ku**
**Kyoto, Kyoto 610-11 (JP)**

(74) Representative : **Bentham, Stephen**
**J.A. KEMP & CO.**
**14 South Square**
**Gray's Inn**
**London WC1R 5LX (GB)**

## (54) Prostaglandin F esters

(57) Prostaglandin F esters of formula (I) :

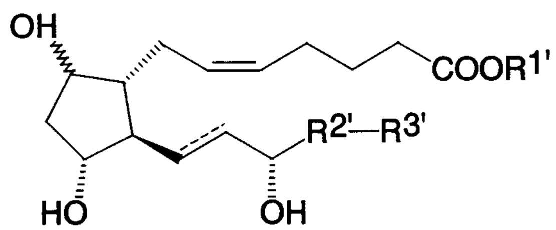

(I)

wherein $R^{1'}$ is a $C_{1-6}$ alkyl, a $C_{4-7}$ carbocycle, or a $C_{1-4}$ alkyl substituted by a $C_{4-7}$ carbocycle, the $C_{4-7}$ carbocycle being optionally substituted by one or more groups independently selected from $C_{1-4}$ alkyl, $C_{1-4}$ alkoxy, halogen, nitro and trifluoromethyl ;

$R^{2'}$ is a bond or $C_{1-4}$ alkylene ;

$R^{3'}$ is $C_{1-7}$ alkyl ; and

the 9-hydroxy group is $\alpha$ or $\beta$ ;

with the provisos that :

(i) when the 9-hydroxy group is $\alpha$ then :

(a) $R^{2'}$-$R^{3'}$ is not n-pentyl ; and

(b) when $R^{1'}$ is methyl and the 13 and 14 positions are singly bonded or when $R^{1'}$ is ethyl and the 13 and 14 positions are doubly bonded, then $R^{2'}$-$R^{3'}$ is not 1,1-dimethylpentyl ; and

(ii) when the 9-hydroxy group is $\beta$,

(a) when $R^{1'}$ is ethyl and the 13 and 14 positions are doubly bonded then $R^{2'}$-$R^{3'}$ is not n-pentyl or 1,1-dimethylpentyl ; and

(b) when $R^{2'}$-$R^{3'}$ is n-pentyl and the 13 and 14 positions are singly bonded then $R^{1'}$ is not $C_{1-6}$ alkyl ;

EP 0 686 628 A2

or a cyclodextrin clathrate thereof, possess ocular hypotensive activity at low concentration and low stimulus and are therefore useful for preventing and /or treating glaucoma.

Processes for their preparation and the use of 16,16-dimethyl PGF2β ethyl ester in the treatment of glaucoma are also disclosed.

The present invention relates to prostaglandin F (abbreviated hereafter as PGF) esters, processes for preparing them and their use as ocular hypotensive agents.

Glaucoma is a disease caused by the elevation of intraocular pressure. If this condition is not alleviated it can cause contraction of the visual field and ultimately blindness.

Intraocular pressure is changed by the balance of the secretion of aqueous humor from the ciliary body epithelium and its excretion from Schlemm's canal. Imbalance causes the elevation of intraocular pressure.

Treatment of glaucoma is currently carried out by two different methods, i.e. by surgery or by the administration of an ocular hypotensive agent. As ocular hypotensive agents, miotic agents (pilocarpine, carbacol etc.), carbonic anhydrase inhibitors and β-blockers are known.

It has become known recently that certain derivatives of prostaglandins (PG) possess ocular hypotensive activity without severe side effects.

For example, it has been disclosed that PGF2α alkyl esters of formula (A) :

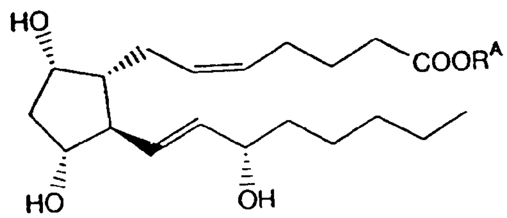

(A)

wherein $R^A$ is C1-5 alkyl (above formula is summarized by the present inventors), possess ocular hypotensive activity (see EP-A-93,380). PGF2α methyl ester, PGF2α ethyl ester, PGF2α isopropyl ester are specifically disclosed therein as effective PGF type compounds. PGF1α, PGF2α, PGF2α tromethamine salt, PGF2β tromethamine salt, PGF2β and 16,16-dimethyl-PGF2α are specifically disclosed as comparative examples.

It has also been disclosed that 11-epi-PGF2α alkyl and alkyl-aryl esters of formula (B) :

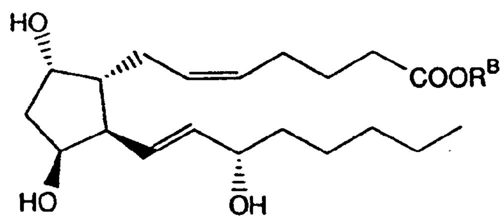

(B)

wherein $R^B$ is alkyl or alkyl-aryl (above formula is summarized by the present inventors), possess ocular hypotensive activity (see EP-A-344,235). 11-epi-PGF2α isopropyl ester is specifically disclosed as an effective compound.

Further, the following compounds having chemical structures partially similar to the compounds of the present invention are known (the numbers in parentheses show CAS registry numbers).

13,14-dihydro-PGF2α methyl ester (69170-52-1)
PGF2α cyclohexyl ester (57893-60-4)
PGF2α phenyl ester (74973-23-2)
PGF2α benzyl ester (71845-64-2)
PGF2β ethyl ester (62791-46-2)
16,16-dimethyl-PGF2α ethyl ester (71098-61-8)
16,16-dimethyl-13,14-dihydro-PGF2α methyl ester (67788-51-6)
16,16-dimethyl-PGF2β ethyl ester (71098-62-9)

However, no ocular hypotensive activity has been disclosed or suggested for any of these compounds.

The present inventors have synthesized many kinds of PGF type compounds and investigated their pharmacological activities and physical properties, and have surprisingly found that certain PGF esters (see formula (I) below) possess strong ocular hypotensive activity, have low side effects and good physical properties.

The distinguishing structural characteristics of the compounds of the present invention are:

(1) the carboxy moiety is modified into an ester; and
(2) the omega-chain is modified;
over naturally occurring PGF.

The compounds of formula (I) as defined below do not include compounds of formula (A) or (B), since these compounds have structures wherein the 9-hydroxy group is attached in α-configuration, the 13 and 14 positions are double bonded and they have an n-pentyl group.

16,16-dimethyl-PGF2β ethyl ester is known and disclosed in US-A-4206127 only as an intermediate for chemical synthesis. Since there is no indication of any activity, nor any indication specifically of activity in reducing intraocular pressure, the present invention includes this compound for use in a method of treatment for the human or animal body.

The present invention provides prostaglandin F esters of formula (I) :

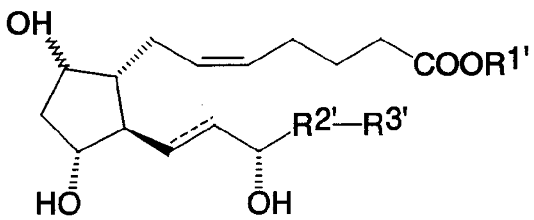

(I)

wherein $R^{1'}$ is a $C_{1-6}$ alkyl, a $C_{4-7}$ carbocycle, or a $C_{1-4}$ alkyl substituted by a $C_{4-7}$ carbocycle, the $C_{4-7}$ carbocycle being optionally substituted by one or more groups independently selected from $C_{1-4}$ alkyl, $C_{1-4}$ alkoxy, halogen, nitro and trifluoromethyl;

$R^{2'}$ is a bond or $C_{1-4}$ alkylene;

$R^{3'}$ is $C_{1-7}$ alkyl; and

the 9-hydroxy group is α or β;

with the provisos that:

(i) when the 9-hydroxy group is α then:

(a) $R^{2'}$-$R^{3'}$ is not n-pentyl; and

(b) when $R^{1'}$ is methyl and the 13 and 14 positions are singly bonded or when $R^{1'}$ is ethyl and the 13 and 14 positions are doubly bonded, then $R^{2'}$-$R^{3'}$ is not 1,1-dimethylpentyl; and

(ii) when the 9-hydroxy group is β,

(a) when $R^{1'}$ is ethyl and the 13 and 14 positions are doubly bonded then $R^{2'}$-$R^{3'}$ is not n-pentyl or 1,1-dimethylpentyl; and

(b) when $R^{2'}$-$R^{3'}$ is n-pentyl and the 13 and 14 positions are singly bonded then R1' is not $C_{1-6}$ alkyl;

or a cyclodextrin clathrate thereof.

The present invention therefore includes:

1) PGF esters of formula (Ia) :

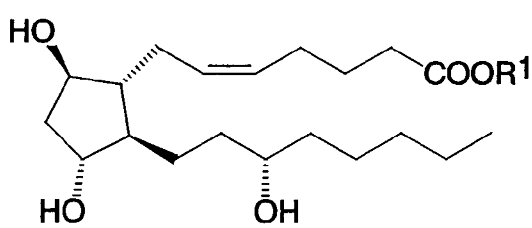

(Ia)

wherein $R^1$ is a $C_{4-7}$ carbocycle or $C_{1-4}$ alkyl which is substituted by a $C_{4-7}$ carbocycle; the $C_{4-7}$ carbocycle being optionally substituted by $C_{1-4}$ alkyl, $C_{1-4}$ alkoxy, halogen, nitro or trifluoromethyl,

2) Prostaglandin F esters of formula (VI) :

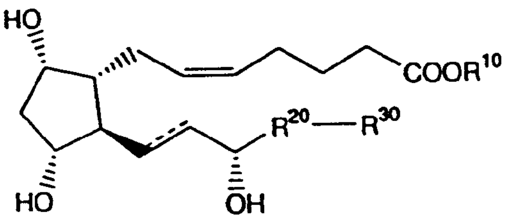

(VI)

wherein $R^{10}$ is a $C_{1-6}$ alkyl, $C_{4-7}$ carbocycle or $C_{1-4}$ alkyl which is substituted by a $C_{4-7}$ carbocycle; $R^{20}$ is a bond or $C_{1-4}$ alkylene; $R^{30}$ is a $C_{1-7}$ alkyl; the $C_{4-7}$ carbocycle being optionally substituted by $C_{1-4}$ alkyl, $C_{1-4}$ alkoxy, halogen, nitro or trifluoromethyl; with the provisos that $R^{20}$-$R^{30}$ is not n-pentyl; and when $R^{10}$ is methyl and the 13 and 14 position are singly bonded or when $R^{10}$ is ethyl and the 13 and 14 positions are doubly bonded, then $R^{20}$-$R^{30}$ is not 1,1-dimethylpentyl,

3) Prostaglandin F esters of formula (XIV) :

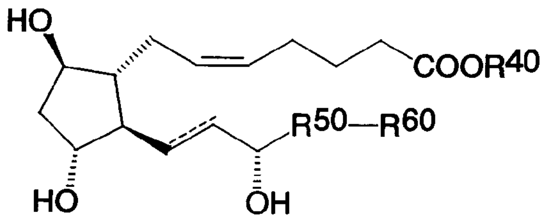

( XIV )

wherein $R^{40}$ is a $C_{1-6}$ alkyl, $C_{4-7}$ carbocycle or $C_{1-4}$ alkyl which is substituted by a $C_{4-7}$ carbocycle; $R^{50}$ is a bond or $C_{1-4}$ alkylene; $R^{60}$ is a $C_{1-7}$ alkyl; the $C_{4-7}$ carbocycle being optionally substituted by $C_{1-4}$ alkyl, $C_{1-4}$ alkoxy, halogen, nitro or trifluoromethyl; with the provisos that $R^{50}$-$R^{60}$ is not n-pentyl; and when $R^{10}$ is ethyl, then $R^{50}$-$R^{60}$ is not 1,1-dimethylpentyl.

$C_{4-7}$ carbocycle means e.g., cyclobutyl, cyclopentyl, cyclohexyl, cycloheptyl, cyclopentadienyl, cyclopentenyl, phenyl, cycloheptatrienyl, cycloheptadienyl, cycloheptenyl etc.

$C_{1-4}$ alkyl means methyl, ethyl, propyl, butyl and isomers thereof.

$C_{1-4}$ alkoxy means methoxy, ethoxy, propoxy, butoxy and isomers thereof.

$C_{1-6}$ alkyl means methyl, ethyl, propyl, butyl, pentyl, hexyl and isomers thereof.

$C_{1-4}$ alkylene means methylene, ethylene, trimethylene, tetramethylene and isomers thereof. 1,1-dimethylmethylene is more preferable.

$C_{1-7}$ alkyl means methyl, ethyl, propyl, butyl, pentyl, hexyl, heptyl and isomers thereof.

It is to be understood that branched isomers of the above alkyl, alkoxy, and alkylene groups are included in the present invention.

Preferred are compounds in which $R^{1'}$ is cyclohexyl, phenyl, benzyl or isopropyl. Also preferred are compounds in whch $R^{2'}$-$R^{3'}$ is n-pentyl or 1,1-dimethylpentyl.

Particularly preferred are compounds in which $R^1$ is isopropyl when the 9-hydroxy group is $\alpha$; and compounds in which the 9-hydroxy group is $\beta$.

Preferred compounds of the present invention are:

13,14-dihydro-PGF2$\beta$ cyclohexyl ester;
13,14-dihydro-PGF2$\beta$ phenyl ester;
13,14-dihydro-PGF2$\beta$ benzyl ester;
16,16-dimethyl-13,14-dihydro-PGF2$\alpha$ isopropyl ester;
16,16-dimethyl-PGF2$\alpha$ isopropyl ester; or
16,16-dimethyl-PGF2$\beta$ isopropyl ester.

Cyclodextrin clathrates of PGF esters of formula (I) and (Ia) may be prepared by the method described in Japanese Patent Kokoku No. 50-3363, ibid. 52-31404 or ibid. 61-52146 i.e. GB-A-1,351,238 or GB-A-1,419,221, using $\alpha$-, $\beta$- or $\gamma$- cyclodextrins or mixture thereof. Converting into their cyclodextrin clathrates serves to increase the stability and solubility in water, and therefore facilitates their administration as pharmaceuticals.

The present invention also provides prostaglandin F esters of fomula (Ib) :

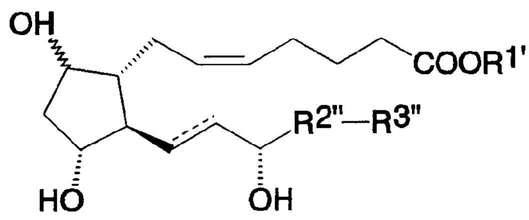

(I b)

wherein $R^{1''}$ is a $C_{1-6}$ alkyl, a $C_{4-7}$ carbocycle or a $C_{1-4}$ alkyl substituted by a $C_{4-7}$ carbocycle, the $C_{4-7}$ carbocycle being optionally substituted by one or more groups independently selected from $C_{1-4}$ alkyl, $C_{1-4}$ alkoxy, halogen, nitro and trifluoromethyl:

$R^{2''}$ is a bond or $C_{1-4}$ alkylene;

$R^{3''}$ is $C_{1-7}$ alkyl; and

the 9-hydroxy group is $\alpha$ or $\beta$;

with the provisos that:

(i) when the 9-hydroxy group is $\alpha$ then:

(a) $R^{2''}$-$R^{3''}$ is not n-pentyl; and

(b) when $R^{1''}$ is methyl and the 13 and 14 positions are singly bonded or when $R^{1''}$ is ethyl and the 13 and 14 positions are doubly bonded, then $R^{2''}$-$R^{3''}$ is not 1,1-dimethylpentyl; and

(ii) when the 9-hydroxy group is $\beta$,

(a) when $R^{1''}$ is ethyl and the 13 and 14 positions are doubly bonded then $R^{2''}$-$R^{3''}$ is not n-pentyl; and

(b) when $R^{2''}$-$R^{3''}$ is n-pentyl and the 13 and 14 positions are singly bonded then $R^{1''}$ is not $C_{1-6}$ alkyl;

or a cyclodextrin clathrate thereof; for use in a method of treatment of the human or animal body.

The compounds of the present invention of formula (Ia) may be prepared by hydrolyzing a compound of formula (II) :

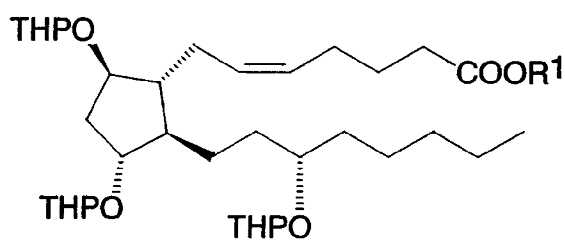

( II )

wherein THP is tetrahydropyran-2-yl, and $R^1$ has the same meaning as hereinbefore described, under acidic conditions.

Hydrolysis under acidic conditions is known. It may be carried out, for example, in water-miscible organic solvent (tetrahydrofuran, dioxan, methanol, ethanol etc.) using acid (acetic acid, trichloroacetic acid, hydrochloric acid, oxalic acid etc.), at a temperature of 0-90°C.

Compounds of formula (II) may be prepared by the following reaction scheme (A).

In the reaction scheme (A), THP is tetrahydropyran-2-yl;

DHP is 3,4-dihydro-2H-pyran;

CSA is camphorsulfonic acid;

THF is tetrahydrofuran;

DMAP is 4-methylaminopyridine.

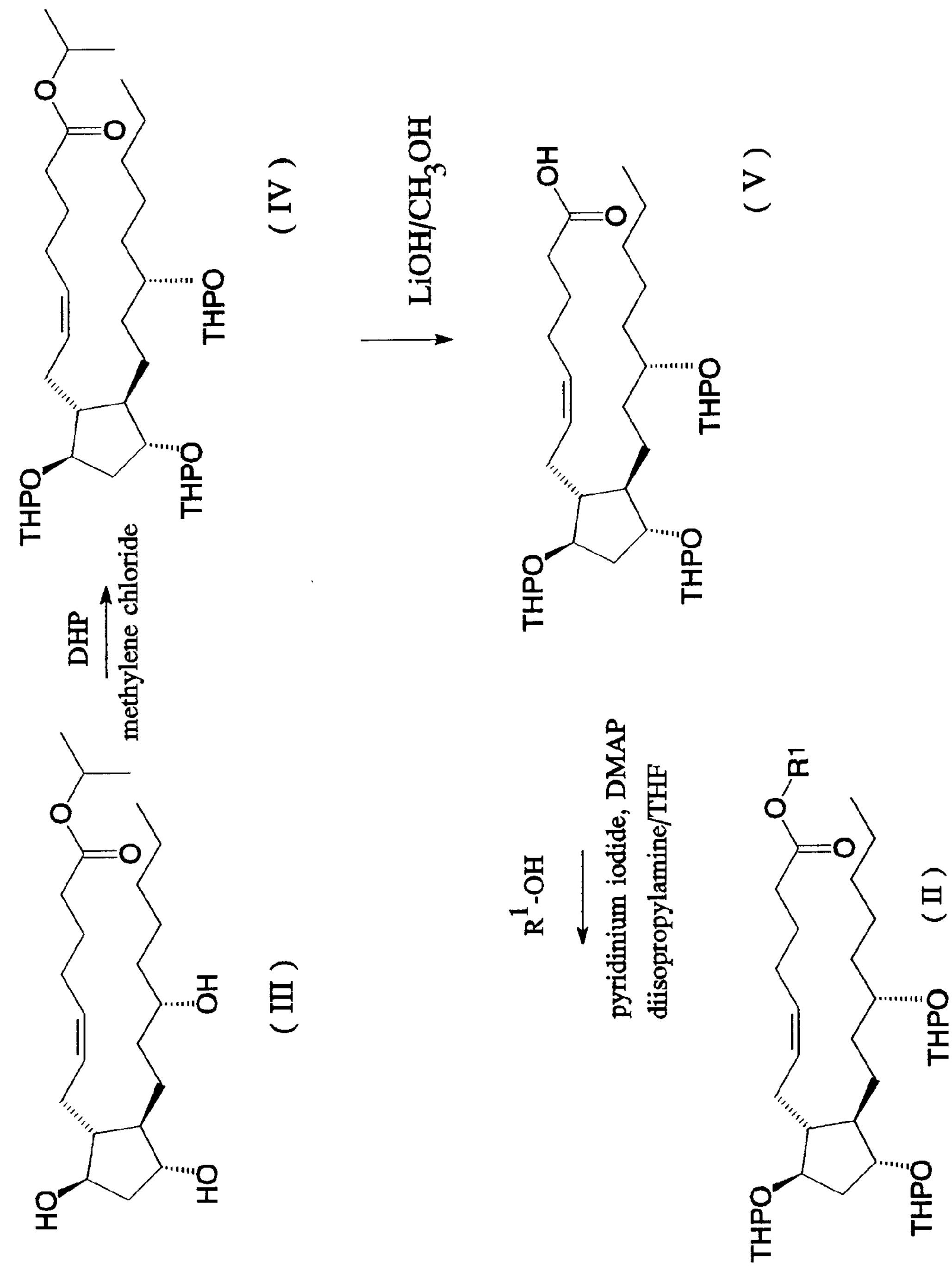


Scheme ( A )

The starting material of formula (III) is a known compound (see EP-A-628,545).

Among the compounds of the present invention of formula (VI), are compounds wherein the 13 and 14 positions are single bonded, of formula (VIa) :

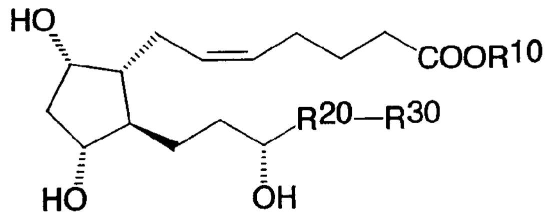

( VIa )

wherein the various symbols have the same meanings as hereinbefore defined, which may be prepared by hydrolyzing a compound of formula (XII) :

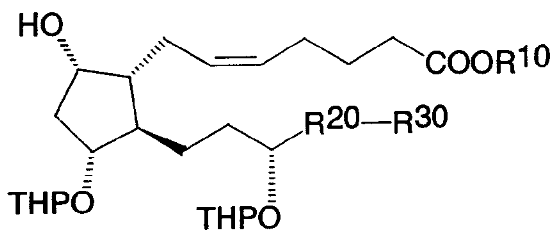

( XII )

wherein the various symbols have the same meanings as hereinbefore defined under acidic conditions.

The hydrolysis under acidic conditions may be carried out by the method hereinbefore described.

Compounds of formula (XII) may be prepared by the following reaction scheme (B).

In the reaction scheme (B), DIBAL is diisobutylaluminum hydride and the other symbols are the same meanings as hereinbefore described.

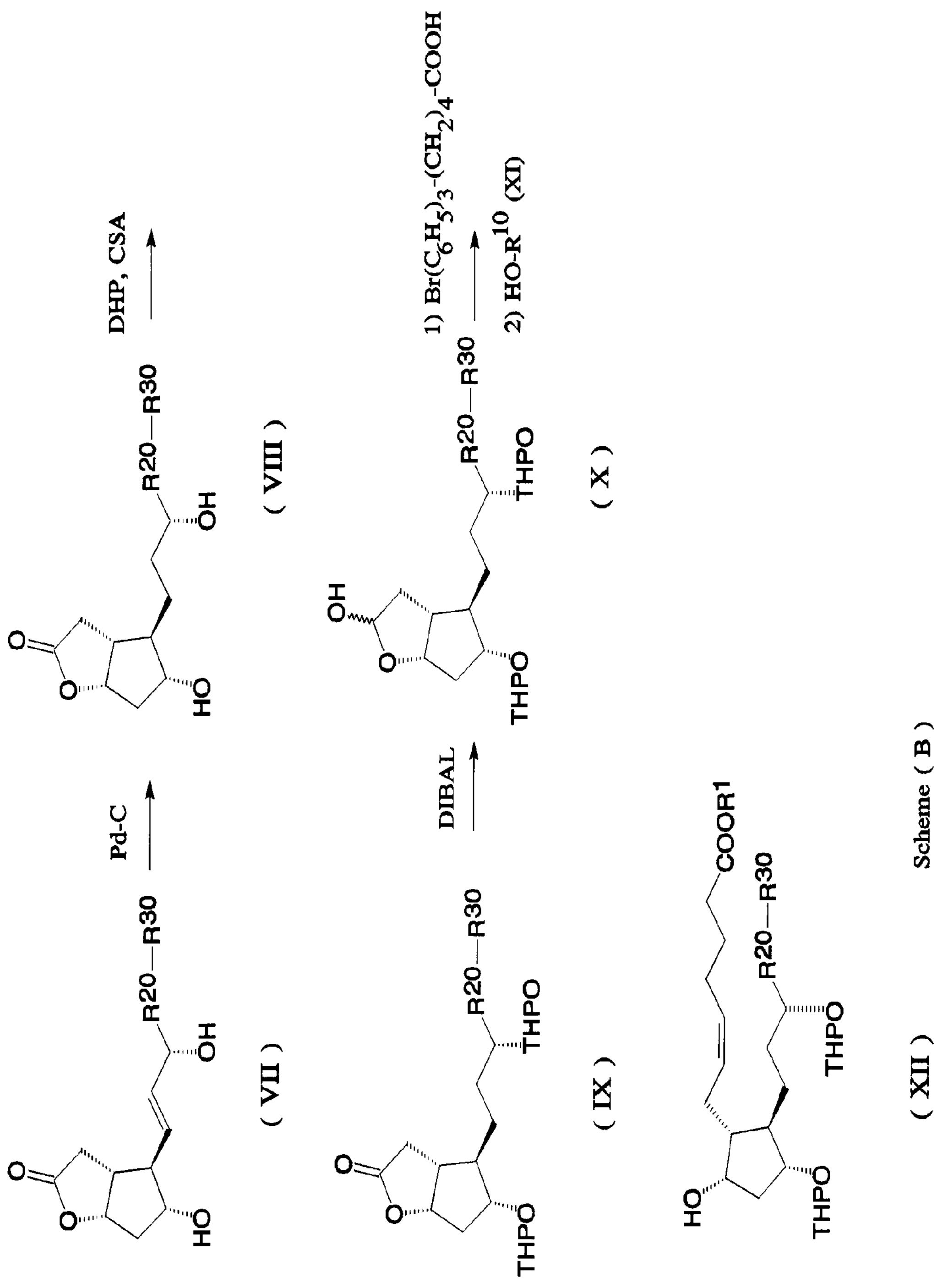


( VII ) ( VIII ) ( IX ) ( X ) ( XII )

Scheme ( B )

Compounds of formula (VII) are known.

Compounds of the present invention of formula (VI) wherein the 13 and 14 positions are double bonded, are of formula (VIb) :

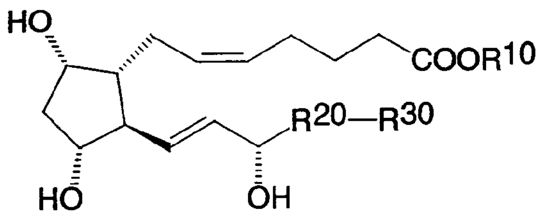

( VIb )

wherein the various symbols have the same meanings as -hereinbefore defined, may be prepared by esterification of a compound of formula (XIII) :

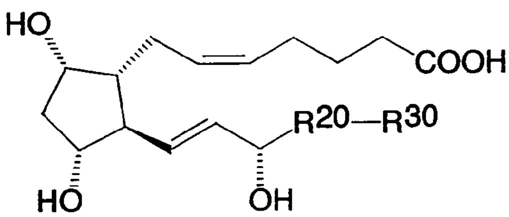

( XIII )

wherein the various symbols have the same meanings as hereinbefore defined.

The esterification is known, for example, by using an alcohol of formula (XI) :

$$HO\text{-}R^{10} \qquad (XI)$$

wherein $R^{10}$ has the same meaning as hereinbefore defined in the presence of 2-chloro-N-methylpyridinium iodide and 4-dimethylaminopyridine at 0-60°C in an inert organic solvent (e.g., tetrahydrofuran, tetrahydropyran etc.).

Compounds of formula (XIII) are known.

Compounds of the present invention of formula (XIV) are prepared by hydrolyzing compounds of formula (XIX) :

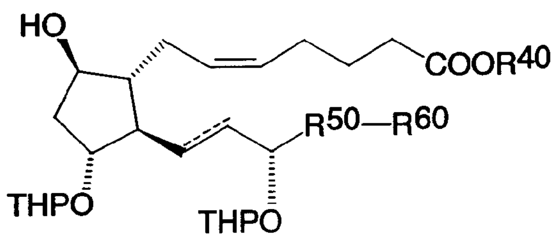

( XIX )

wherein the various symbols have the same meanings as hereinbefore defined, under acidic conditions.

The hydrolysis under acidic conditions may be carried out by the method hereinbefore described.

Compounds of formula (XIX) may be prepared by the-following reaction scheme (C).

In the reaction scheme (C), the symbols have the same meanings as hereinbefore described.

Scheme (C)

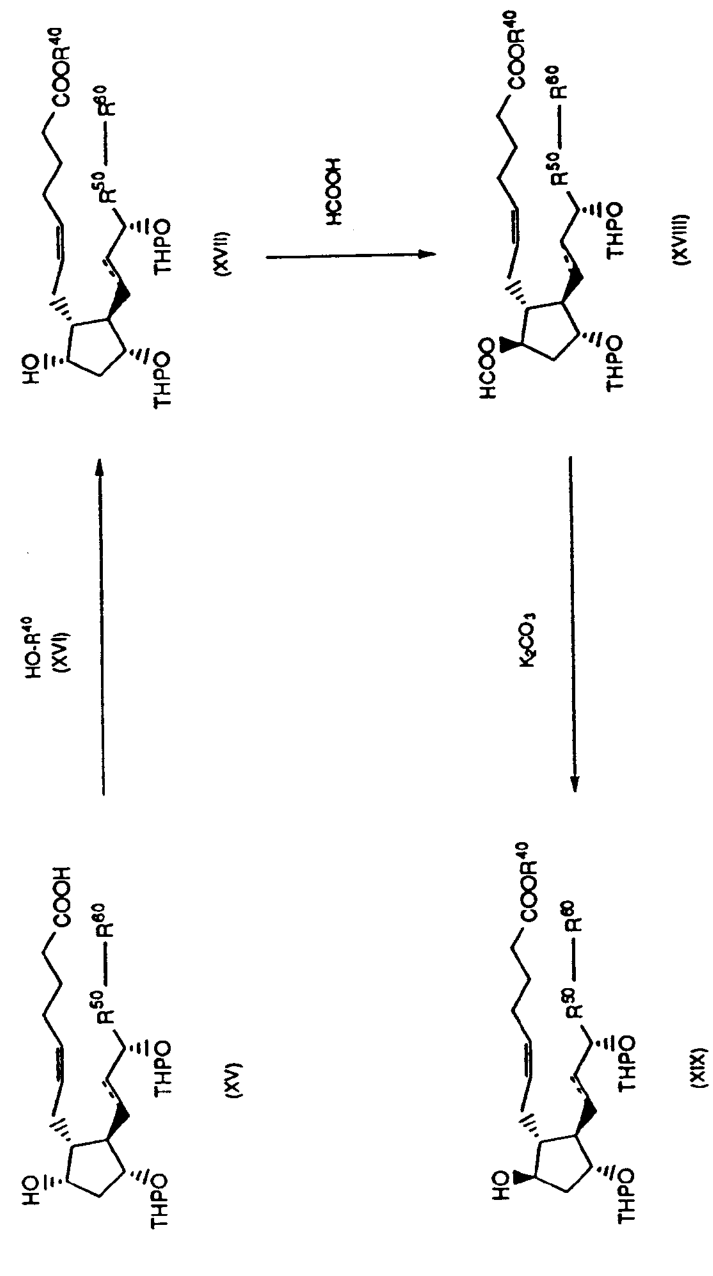


Compounds of formula (XV) are known.

The compounds of the present invention possess ocular hypotensive activity as described before. For example, in a standard laboratory test the following data shown in Table 1 and Table 2 and in the accompanying Fig.1, Fig.2 and Fig.3 were obtained. Each data represents the mean of three or four samples.

Experiment 1

Male Japanese White rabbits weighing about 3.0 kg were lightly held and the intraocular pressure was measured by Applanation Pneumatonograph after corneal anesthesia with instillation of 0.4% oxybuprocaine hydrochloride solution.

A test compound was dissolved at 0.1% in vehicle which contains 2.5% glycerol and 2.0% polysorbate 80 in sterile purified water. The test solution at volume of 50 ml was instilled into the left eye of each rabbit and the right eye remained untreated. As a control, the vehicle was instilled into the left eye in a control group. Δ IOP (mmHg), which is an index of ocular hypotensive effect, were calculated using below formula.

$$\Delta \text{IOP} = (\text{IOPt} - \text{IOP0}) - (\text{IOPvt} - \text{IOPv0})$$

IOPt : IOP at t hr. after instillation of a test solution

IOPvt : IOP at t hr. after instillation of vehicle
IOP0 : IOP just before instillation of a test solution
IOPv0 : IOP just before instillation of vehicle

Result

The graphs of results that a 0.1% solution of the compound of the present invention reduced intraocular pressure are shown in Figure 1 and Figure 2.

As shown in Figure 1 and Figure 2, a 0.1% solution of the compound of the present invention reduced intraocular pressure immediately after the instillation. For example, the maximum reduction of IOP after instillation of the compounds shown as examples 1, 1(a), 1(b), 2, 3, 4 and 5 hereinafter were 6.0, 5.7, 5.4, 4.0, 4.7, 6.7 and 9.8 mmHg, respectively. And when the compound of the present invention was instilled, bulbar and palpebral conjunctival hyperemia could hardly be observed.

These results showed that the compound of the present invention had a strong and long lasting ocular hypotensive effect. The compound of the present invention is excellent and effective enough for pharmaceutical use, free from side-effects, with desirable and physical properties.

Further, the compounds of the present invention were compared with the compounds shown concretely in related arts hereinbefore described. The compounds have chemical structures quite similar to the compounds of the present invention were selected as compared compounds. 0.02% solutions of the compounds of the present invention were compared with the compared compounds, because the 0.02% solution is close to concentration used actually.

The compared compounds 1 and 2 shown in Table 1 and Table 2 and in Figure 1 and Figure 2 hereinafter shown are the following:

compared compound 1---- 13,14-dihydro-PGF2α cyclohexyl ester which is described in example 3 of Japanese Patent Kokai No. 50-14660

compared compound 2---- 16,16-dimethyl-PGF2α which is described in the 3rd Table of Japanese Patent Kokai No. 59-1418 i.e. European Patent No. 344,235

Experiment 2

The compounds of the present invention and compared compounds were dissolved in vehicle at 0.02%, and Δ IOPs (mmHg) after instillation of these solution, which are the index of ocular hypotensive effects, were calculated in the same way as the experiment 1.

Result

The results that a 0.02% solution of the compound of the present invention reduced intraocular pressure in comparison with compared compound were shown in Table 1 and Table 2. The graphs of the results shown in the Table 1 and Table 2 were Figure 1 and Figure 2.

Table 1: Ocular Hypotensive Effects on Intraocular Pressure

| Time after Administration (hr.) | 0 | 1 | 2 | 4 | 6 |
|---|---|---|---|---|---|
| Example 1 | 0 | -2.0 | -2.3 | -6.6 | -1.8 |
| Compared compound 1 | 0 | 11.3 | 2.3 | -5.6 | -9.3 |

(mmHg)

Table 2: Ocular Hypotensive Effects on Intraocular Pressure

| Time after Administration (hr.) | 0 | 1 | 2 | 4 | 6 |
|---|---|---|---|---|---|
| Example 4 | 0 | -5.0 | -5.0 | -12.3 | -9.6 |
| Example 5 | 0 | -2.0 | -3.5 | -7.3 | -7.3 |
| Compared compound 2 | 0 | 4.5 | 4.5 | -2.0 | -0.8 |

(mmHg)

Discussion

As an anti-glaucoma agent, following properties are required:

1) rapid onset and long lasting ocular hypotensive effect,

2) less side effects such as initial elevation of intraocular pressure.

We compared 0.02% solution of the compound of the present invention (example 1) with compared compounds 1, and the compound of the present invention (examples 4 and 5) with compared compound 2 from the above view points.

First, comparison is done from the view point 1). The compound of the present invention (example 1) showed:

a. The ocular hypotensive effect appeared at 1 hr. after administration.

b. The activity reached 6.6 mmHg at 4 hrs. after administration and same level activity was maintained for 6 hrs. after administration.

Compared compound 1 showed:

a. The effect appeared barely at 4 hrs. after administration.

Next, comparison is done from the view point 2). The compound of the present invention (example 1) did not show initial elevation of the intraocular pressure. The compared compound 1 showed extremely strong initial elevation. From the view point of the initial elevation, it is clear that the compound of the present invention is superior to these compared compounds.

The compound of the present invention (examples 4 and 5) showed concerning the view point 1):

a. The effect appeared at 1 hr. after administration.

b. The activity was maintained for 6 hrs. after administration.

Compared compounds 2 showed:

a. The effect appeared barely at 4 hrs. after administration.

Concerning the view point 2), the compared of the present invention (examples 4 and 5) did not show initial elevation of the intraocular pressure. The compared compound 2 showed initial elevation and lasted for 4 hrs.

The toxicity of the compounds of the present invention is very low and therefore, it may be confirmed that the compounds of the present invention are safe for pharmaceutical use.

The reduction of ocular pressure is an effective method for prevention and/or treatment of glaucoma in animals including human beings, especially human beings.

The compounds of the present invention possess the activity of reducing intraocular pressure without side effects such as stimulus to eyes, from the results of in vivo experiment, so they are effective for the prevention and/or treatment of glaucoma.

For the purpose above described, the compound of the present invention of the formula (I), may be used normally as a solution. The solutions may be prepared by normal techniques.

For example, eye drops may be prepared by adding to sterilized and purified water, isotonic buffer (sodium chloride, concentrated glycerol etc.), buffer (sodium phosphate etc.), dissolving agent (Polysorbate 80 etc.), stabilizing agent (sodium edetate etc.) and/or preserving agent (benzalkonium chloride etc.) etc. for necessary.

The doses to be administered in the form of eye drops are determined depending upon symptoms, age, the desired therapeutic effects, the duration of the treatment etc. As normally used, the concentration of the solution is between 0.0001% and 0.5%. A preferable concentration of the solution is between 0.001% and

0.2%.

The following reference examples and examples illustrate the present invention. The solvents in the parentheses show the developing or eluting solvents and the ratios of the solvents used are by volume in chromatographic separation.

Unless otherwise specified, "NMR" was measured in chloroform-d.

Reference example 1

Synthesis of 9β,11α,15α-tris(2-tetrahydropyranyloxy)-5Z-prostenoic acid isopropyl ester

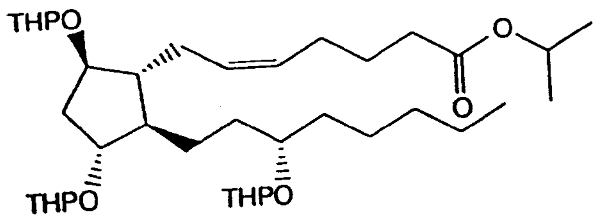

13,14-dihydro-PGF2β isopropyl (243 mg ; a compound of example 1 in Japanese Patent Application No. 5-167531 i.e. European Patent Publication No. 628,545) was dissolved into anhydrous methylene chloride (15 ml). 1,4-dihydropyran (0.33 ml) and dl-camphorsulfonic acid (23 mg) were added to the solution. The mixture was stirred for 3 hrs. at room temperature under an atmosphere of argon. Triethylamine (0.2 ml) was added to the reaction mixture and the mixture was evaporated. The residue was purified by column chromatography on silica gel (ethyl acetate / hexane) to give the title compound (390 mg) having the following physical data:
Appearance : colorless oil;
TLC : Rf 0.84 (ethyl acetate : hexane = 10 : 1).

Reference example 2

Synthesis of 9β,11α,15α-tris(2 tetrahydropyranyloxy)-5Z-prostenoic acid

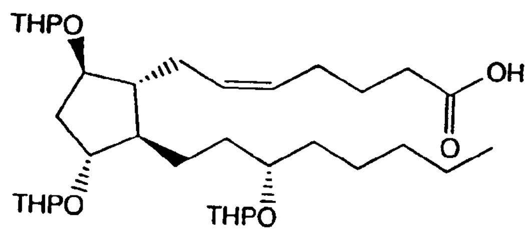

The compound (390 mg) prepared in reference example 1 was dissolved into methanol (15ml). Lithium hydride monohydrate (252 mg) was dissolved into water (3 ml) and this solution was added to the above solution in methanol. The reaction mixture was stirred for 4 hrs. at 50°C. After the reaction, ethyl acetate (30 ml) was added to the reaction mixture and the mixture was washed with a solution of oxalic acid monohydrate (454 mg) in water (30 ml) and a saturated aqueous solution of sodium chloride, successively. The washed solution was extracted with ethyl acetate and the extract was added to the organic layer. The organic layer was dried over anhydrous magnesium sulfate, and evaporated. The residue was purified by column chromatography on silica gel (ethyl acetate / hexane) to give the title compound (359 mg) having the following physical data:
Appearance : colorless oil;
TLC : Rf 0.42 (ethyl acetate : hexane = 10 : 1).

Reference example 3

Synthesis of 9β,11α,15α-tris (2-tetrahydropyranyloxy) -5Z-prostenoic acid cyclohexyl ester

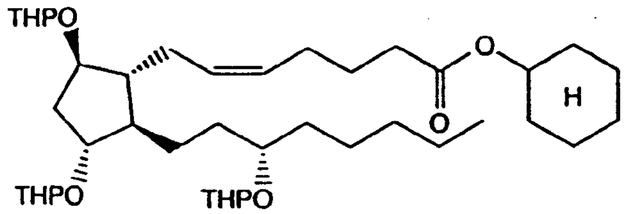

The compound (61 mg) prepared in reference example 2 was dissolved into anhydrous tetrahydrofuran (5 ml). Cyclohexanol (21 μl), 2-chloro-N-methylpyridinium iodide (51 mg), diisopropylethylamine (70 μl) and 4-dimethylaminopyridine (6.1 mg) were added to the solution. The reaction mixture was stirred for 4 hrs. at room temperature under an atmosphere of argon. After the reaction, ethyl acetate was added to the reaction mixture. The mixture was washed with 1N hydrochloric acid, a saturated aqueous solution of potassium hydrocarbonate and a saturated aqueous solution of sodium chloride,
successively, dried over anhydrous magnesium sulfate and evaporated. The residue was purified by column chromatography on silica gel (ethyl acetate / hexane) to give the title compound (37 mg) having the following physical data:
Appearance : colorless oil;
TLC : Rf 0.85 (ethyl acetate : hexane = 10 : 1).

Example 1

Synthesis of 13,14-dihydro-PGF2β cyclohexyl ester

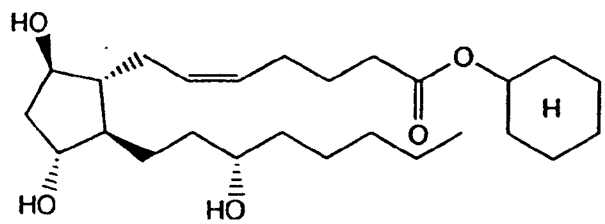

A mixture of 65% acetic acid (5 ml) and tetrahydrofuran (0.5 ml) was added to the compound (37 mg) prepared in reference example 3. The mixture was stirred for 3 hrs. at 50°C. After the reaction, the reaction mixture was cooled to room temperature and diluted with ethyl acetate (20 ml). The solution was washed with a saturated aqueous solution of potassium hydrocarbonate and a saturated aqueous solution of sodium chloride, successively and evaporated. The residue was purified by column chromatography on silica gel (ethyl acetate / hexane) to give the title compound (19 mg) having the following physical data:
Appearance : pale yellow crystal;
TLC : Rf 0.41 (methanol : methylene chloride = 1 : 10);
NMR : δ 5.60-5.38 (2H, m), 4.78 (1H, m), 4.10 -3.95 (2H, m), 3.65-3.50 (1H, br), 2.60-1.10 (37H, m), 0.88 (3H, t).

Example 1(a)-(b)

By the same procedure as a series of reactions of reference examples 1-3 and example 1, the compounds having the following physical data were given respectively.

Example 1(a)

Synthesis of 13,14-dihydro-PGF2β phenyl ester

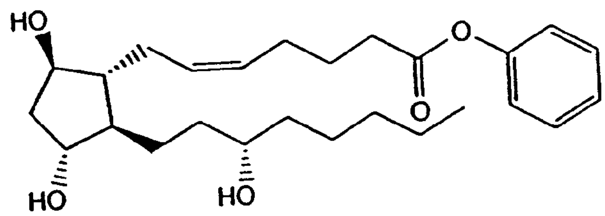

Appearance : colorless crystal;
TLC : Rf 0.28 (methanol : methylene chloride = 1 : 10);
NMR : δ 7.37 (2H, t), 7.21 (1H, t), 7.08 (2H, d), 5.62-5.40 (2H, m), 4.08-3.90 (2H, m), 3.66-3.50 (1H, br), 2.80-1.10 (27H, m), 0.90 (3H, t).

Example 1(b)

Synthesis of 13,14-dihydro-PGF2β benzyl ester

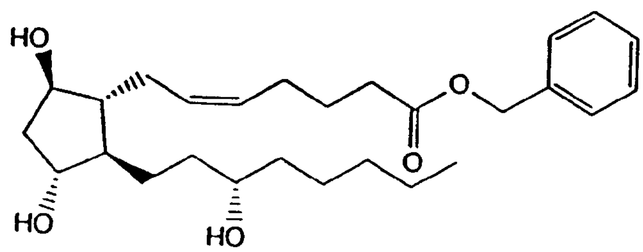

Appearance : pale yellow crystal;
TLC : Rf 0.26 (ethyl acetate : methylene chloride = 1 : 10);
NMR : δ 7.35 (5H, s), 5.60-5.34 (2H, m), 5.10 (2H, s), 4.08-3.95 (2H, m), 3.67-3.52 (1H, br), 2.60-1.20 (27H, m), 0.88 (3H, t).

Reference example 4

Synthesis of 7R-hydroxy-6R-(4,4-dimethyl-3R-hydroxyoctyl)-2-oxabicyclo[3.3.0]ocatan-3-one

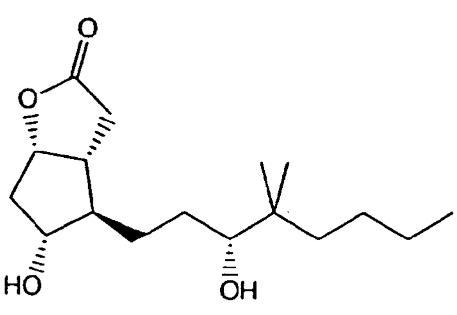

A mixture of 7R-hydroxy-6R-(4,4-dimethyl-3R-hydroxyoct-1-enyl)-2-oxabicyclo[3.3.0]ocatan-3-one (3.25 mg; the compound of the formula (III) in Prostaglandins, 4, 143 (1973), Magerlein et al.), sodium nitrite (325 mg), palladium-carbon (5%; 325 mg) and ethanol (110 ml) was vigorously stirred for 3.5 hrs. at room temperature under an atmosphere of hydrogen. After the reaction, the reaction mixture was filtered with glass filter, washed with ethanol, and evaporated to give the title compound (3.25 g) having the following physical data:
Appearance : colorless oil;
TLC : Rf 0.45 (ethyl acetate : hexane = 9 : 1).

Reference example 5

Synthesis of 7R-(2-tetrahydropyranyloxy)-6R-[4,4-dimethyl-3R-(2-tetrahydropyranyloxy) octyl] -2-oxabicyclo [3.3.0] ocatan-3-one

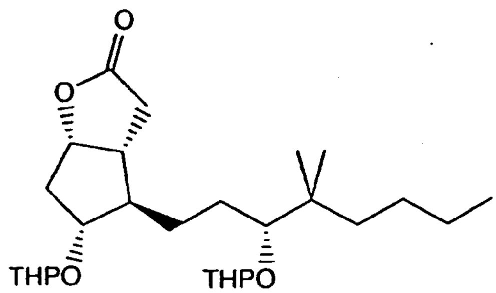

The compound (3.25 g) prepared in reference example 4 was dissolved into methylene chloride (110 ml), and dihydropyran (3 ml) and camphorsulfonic acid (1.16 g) were added to the solution. The reaction solution was stirred for 1 hr. at room temperature. After triethylamine (1 ml) was added to the reaction solution, the mixture was evaporated. The residue was purified by column chromatography on silica gel (ethyl acetate / hexane) to give the title compound (4.34 g) having the following physical data:
Appearance : pale yellow oil;
TLC : Rf 0.65 (ethyl acetate);
NMR : δ 5.00(1H, m), 4.65(1H, m), 4.65-4.35(1H, m), 4.10(1H, m), 4.00-3.73(3H, m), 3.58-3.33(2H, m), 3.32-3.08(1H, m), 2.90-2.35(3H, m), 2.30-1.00(24H, m), 0.88(3H, t), 0.84(3H, s), 0.82(3H, s).

Reference example 6

Synthesis of 7R-(2-tetrahydropyranyloxy)-6R-[4,4-dimethyl-3R-(2-tetrahydropyranyloxy) octyl] -2-oxabicyclo [3.3.0] ocatan-3-ol

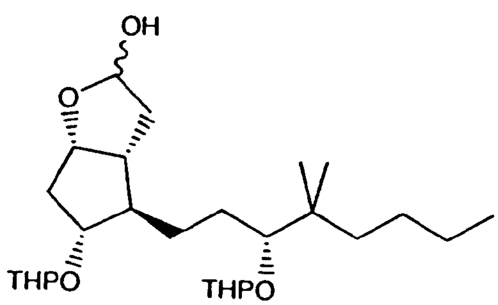

The compound (4.34 g) prepared in reference example 5 was dissolved into anhydrous toluene (60 ml) and the solution was cooled with methanol-dry ice bath. DIBAL (7.44 ml; 1.5 M solution in toluene) was added dropwise to the solution for 8 mins. under an atmosphere of argon. After the reaction, the mixture was vigorously stirred for 20 mins., methanol (0.8 ml) was added dropwise to the mixture and the bath was changed to water bath. Water (0.8 ml) was added dropwise to the reaction mixture and the mixture was vigorously stirred for 1 hr. at room temperature. The reaction mixture was filtered with glass filter under reduced pressure and the impurity was washed with ethyl acetate. The gathered filtrate was evaporated to give the title compound (4.11 g) having the following physical data:
Appearance : colorless crystal;
TLC : Rf 0.26 (ethyl acetate : hexane = 1 : 1);

Reference example 7

Synthesis of 11,15-O-bis(2-tetrahydropyranyl) -16,16-dimethyl-13,14-dihydro-PGF2α

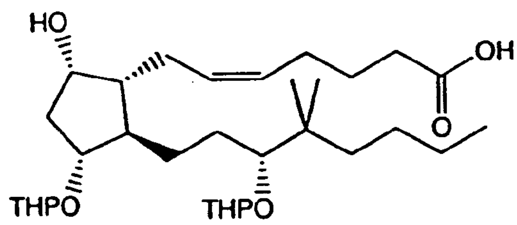

Anhydrous toluene (200 ml) and potassium t-butoxide (10.43 g) were added to dried 4-carboxybutyl-triphenylphosphonium bromide (20.61 g) and the mixture was stirred for 1 hr. at 80°C. After the solution was thoroughly cooled, a solution of the compound (4.11 g) prepared in reference example 6 in anhydrous toluene (37 ml) was added dropwise to the solution for 10 min. The reaction mixture was stirred for 30 min., diluted with ethyl acetate (100 ml) and washed with a aqueous solution of oxalic acid (dihydrate, 7.03g / 150 ml) and a saturated aqueous solution of sodium chloride (150 ml), successively. The organic layer was dried over anhydrous magnesium sulfate and evaporated to give the title compound (18.6 g).

Reference example 8

Synthesis of 11,15-O-bis(2-tetrahydropyranyl)-16,16-dimethyl-13,14-dihydro-PGF2α isopropyl ester

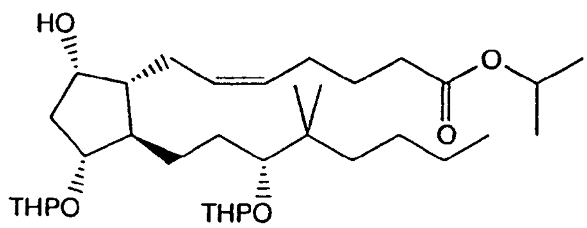

The compound (18.6 g) prepared in reference example 7 was dissolved into anhydrous tetrahydrofuran (200 ml), and isopropyl alcohol (52 ml), diisopropylethylamine (23.6 ml), 2-chloro-N-methylpyridinium iodide (17.4 g) and 4-dimethylaminopyridine (2.06 g) were added to the solution. The reaction mixture was stirred for 1.5 hrs. at room temperature. After the reaction, the reaction mixture was diluted with ethyl acetate (100 ml) and washed with a aqueous solution of oxalic acid (dihydrate, 11.47g / 150 ml), a saturated aqueous solution of potassium hydrocarbonate and a saturated aqueous solution of sodium chloride, successively. The organic layer was dried over anhydrous magnesium sulfate and evaporated. The residue was purified by column chromatography on silica gel (ethyl acetate / hexane) to give the title compound having the following physical data:
Appearance : colorless oil;
TLC : Rf 0.11 (ethyl acetate : hexane = 1 : 5);
NMR : δ 5.60-5.28(2H, m), 5.00(1H, m), 4.75-4.40(2H, m), 4.15-3.75(4H, m), 3.55-3.05(3H, m), 2.60-1.00(34H, m), 1.22(6H, d), 0.95-0.78(9H, m).

Example 2

Synthesis of 16,16-dimethyl-13,14-dihydro-PGF2α isopropyl ester

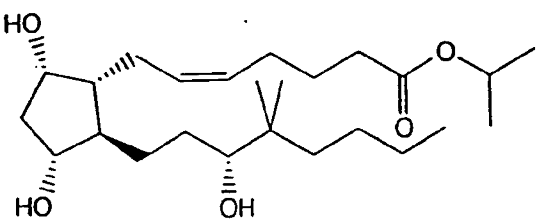

The compound (580 mg) prepared in reference example 8 was dissolved into 65% acetic acid (10 ml) and tetrahydrofuran (1 ml) and the solution was stirred for 4 hrs. at 50°C. The reaction mixture was diluted with

ethyl acetate (50 ml) and washed with a aqueous solution of sodium chloride (30 ml). The organic layer was dried over anhydrous magnesium sulfate and evaporated. The residue was purified by column chromatography on silica gel (ethyl acetate / hexane) to give the title compound (336 mg) having the following physical data:
Appearance : colorless oil;
TLC : Rf 0.52 (ethyl acetate : hexane = 4 : 1);
NMR : δ 5.56-5.30(2H, m), 5.00(1H, m), 4.23-4.10(1H, br), 4.05-3.92(1H, br), 3.35-3.20(1H, br), 2.74-1.10(25H, m), 1.23(6H, d), 0.90(3H, t), 0.86(3H, s), 0.84(3H, s).

Example 3

Synthesis of 16,16-dimethyl-PGF2α isopropyl ester

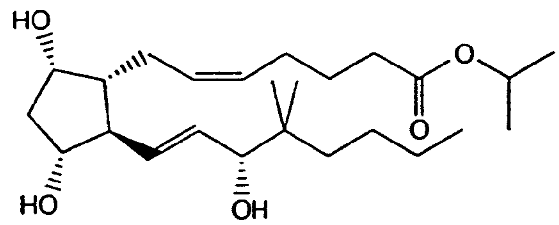

16,16-dimethyl-PGF2α (241 mg; the compound of the formula(V) in Prostaglandins, 4, 143 (1973), B. J. Magerlein et al.) was dissolved into anhydrous tetrahydrofuran (10 ml), and isopropyl alcohol (1.22 ml), isopropylethylamine (0.44 ml), 2-chloro-N-methylpyridinium iodide (322 mg) and 4-dimethylaminopyridine (38 mg) were added to the solution and the mixture was stirred for 3.5 hrs. at room temperature. The reaction mixture was diluted with ethyl acetate (30 ml) and washed with 1N hydrochloric acid cooled with ice, a saturated aqueous solution of potassium hydrocarbonate and a saturated aqueous solution of sodium chloride, successively. The organic layer was dried over anhydrous magnesium sulfate and evaporated. The residue was purified by column chromatography on silica gel (ethyl acetate / hexane) to give the title compound (201 mg) having the following physical data:
Appearance : colorless oil;
TLC : Rf 0.30 (methanol : methylene chloride = 1 : 10); NMR : δ 5.72-5.30(4H, m), 5.00(1H, m), 4.23-4.10(1H, br), 4.05-3.88(1H, br), 3.78(1H, d), 3.18-2.98(1H, br), 2.58-1.00(20H, m), 1.23(6H, d), 0.90(3H, t), 0.88(3H, s), 0.84(3H, s).

Reference example 9

Synthesis of 11,15-O-bis (2-tetrahydropyranyl) -16,16-dimethyl-PGF2α isopropyl ester

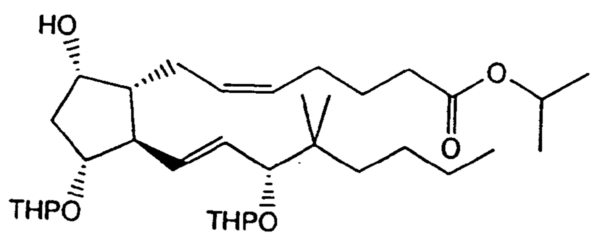

11,15-bis(2-tetrahydropyranyl) -16,16-dimethyl-PGF2α (1.00 g; the compound of the formula (IV) in Prostaglandins, 4, 143 (1973), B. J. Magerlein et al.) was dissolved into anhydrous tetrahydrofuran (20 ml), and isopropyl alcohol (3.6 ml), diisopropylethylamine (1.28 ml), 2-chloro-N-methylpyridinium iodide (940 mg) and 4-dimethylaminopyridine (112 mg) were added to the solution and the mixture was stirred for 3.5 hrs. at room temperature. The reaction mixture was diluted with ethyl acetate (30 ml) and washed with a aqueous solution of oxalic acid (dihydrate, 512 mg / 50 ml), a saturated aqueous solution of potassium hydrocarbonate and a saturated aqueous solution of sodium chloride, successively. The organic layer was dried over anhydrous magnesium sulfate and evaporated. The residue was purified by column chromatography on silica gel (ethyl acetate / hexane) to give the title compound (850 mg) having the following physical data:

Appearance : pale yellow oil;
TLC : Rf 0.55 (ethyl acetate : hexane = 1 : 2).

Reference example 10

Synthesis of 9-O-formyl-11,15-O-bis(2-tetrahydropyranyl)-16,16-dimethyl-PGF2β isopropyl ester

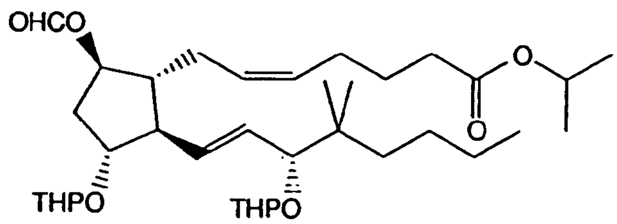

The compound (850 mg) prepared in reference example 9 was dissolved into anhydrous tetrahydrofuran (7 ml), and formic acid (97 μl), triphenylphosphine (674 mg) and diethyl azodicarbonate (0.41 ml) were added to the solution at room temperature under an atmosphere of argon. The reaction mixture was stirred for 1.5 hrs. at room temperature. After the reaction, the mixture was diluted with ethyl acetate and washed with a saturated aqueous solution of potassium hydrocarbonate and a aqueous solution of sodium chloride, successively. The organic layer was dried over anhydrous magnesium sulfate and evaporated. The residue was purified by column chromatography on silica gel (ethyl acetate / hexane) to give the title compound (570 mg) having the following physical data:
Appearance : pale yellow oil;
TLC : Rf 0.31 (ethyl acetate : hexane = 1 : 4).

Reference example 11

Synthesis of 11,15-O-bis(2-tetrahydropyranyl)-16,16-dimethyl-PGF2β isopropyl ester

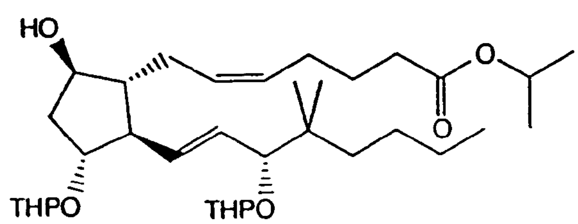

The compound (560 mg) prepared in reference example 10 was dissolved into isopropyl alcohol (4.5 ml), and anhydrous potassium hydrocarbonate (370 mg) was added to the solution. The reaction mixture was stirred for 3 hrs. at 50°C under an atmosphere of argon. After the mixture was cooled diethyl ether (20 ml) was added to the reaction mixture. The mixture was filtered with glass filter and the filtrate was evaporated. The residue was purified by column chromatography on silica gel (ethyl acetate / hexane) to give the title compound (518 mg) having the following physical data:
Appearance : colorless oil;
TLC : Rf 0.45 (ethyl acetate : hexane = 1 : 1).

Example 4

Synthesis of 16,16-dimethyl-PGF2β isopropyl ester

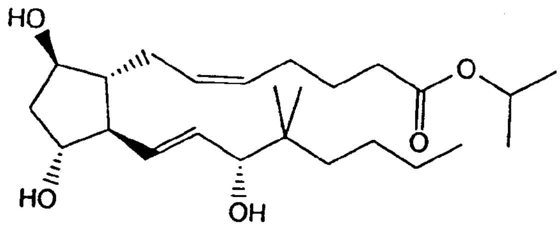

The compound (503 mg) prepared in reference example 11 was dissolved into 65% acetic acid (10 ml) and tetrahydrofuran (1 ml) and the solution was stirred for 4 hrs. at 50°C. The reaction mixture was cooled, diluted with ethyl acetate (50 ml) and washed with a saturated aqueous solution of potassium hydrocarbonate and a aqueous solution of sodium chloride, successively. The organic layer was dried over anhydrous magnesium sulfate and evaporated. The residue was purified by column chromatography on silica gel (ethyl acetate / hexane) to give the title compound (250 mg) having the following physical data:
Appearance : colorless oil;
TLC : Rf 0.26 (methanol : methylene chloride = 1 : 10).
NMR : δ 5.70-5.40(4H, m), 5.00(1H, m), 4.15-3.88(2H, m), 3.78(1H, d), 2.90-2.76(1H, br), 2.34-1.00(3H, t), 0.88(3H, s), 0.84(3H, s).

Example 5

16,16-dimethyl-PGF2β ethyl ester

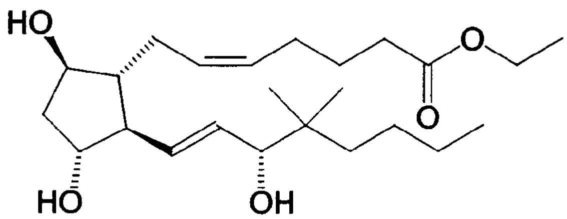

The title compound, preparation thereof and physical characteristics are disclosed in Example 15 of US-A-4206127.

Formulation example

Conc. glycerol and Polysorbate 80 were added to sterilized water. 13,14-dihydro-PGF2β ester was dissolved to the solution to obtain following eye drop solutions.

Table 3:

| Formulation 1 | |
|---|---|
| 13,14-dihydro-PGF2β cyclohexyl ester | 0.001 g |
| Conc. glycerol | 2.5 g |
| Polysorbate 80 | 0.2 g |
| sterilized water | appropriate |
| sum | 100 ml |

Table 4:

| Formulation 2 | |
|---|---|
| 13,14-dihydro-PGF2β cyclohexyl ester | 0.01 g |
| Conc. glycerol | 2.5 g |
| Polysorbate 80 | 0.2 g |
| sterilized water | appropriate |
| sum | 100 ml |

Table 5: Formulation 3

| | |
|---|---|
| 13,14-dihydro-PGF2β cyclohexyl ester | 0.1 g |
| Conc. glycerol | 2.5 g |
| Polysorbate 80 | 0.2 g |
| sterilized water | appropriate |
| sum | 100 ml |

Table 6:

| Formulation 4 | |
|---|---|
| 13,14-dihydro-PGF2β cyclohexyl ester | 0.2 g |
| Conc. glycerol | 2.5 g |
| Polysorbate 80 | 0.2 g |
| sterilized water | appropriate |
| sum | 100 ml |

Brief Description of the Figure

Fig.1 The graph shows ocular hypotensive activity of the compounds of the present invention described in examples 1, 1(a) and 1(b) at 0.1%.

Fig.2 The graph shows ocular hypotensive activity of the compounds of the present invention described in examples 2, 3, 4 and 5 at 0.1%.

Fig.3 The graph shows a ocular hypotensive activity of the compounds of the present invention described in example 1 in comparison with compared compound 1 at 0.02%.

Fig.4 The graph shows a ocular hypotensive activity of the compounds of the present invention described in examples 4 and 5 in comparison with compared compound 2 at 0.02%.

## Claims

1. A prostaglandin F ester of formula (I) :

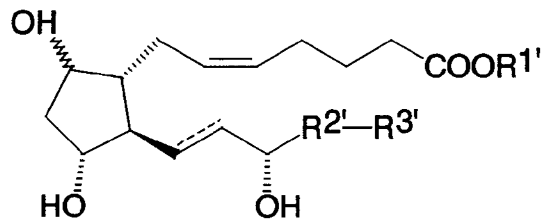

( I )

wherein $R^{1'}$ is a $C_{1-6}$ alkyl, a $C_{4-7}$ carbocycle, or a $C_{1-4}$ alkyl substituted by a $C_{4-7}$ carbocycle, the $C_{4-7}$ carbocycle being optionally substituted by one or more groups independently selected from $C_{1-4}$ alkyl, $C_{1-4}$ alkoxy, halogen, nitro and trifluoromethyl;

$R^{2'}$ is a bond or $C_{1-4}$ alkylene;

$R^{3'}$ is $C_{1-7}$ alkyl; and

the 9-hydroxy group is α or β;

with the provisos that:

(i) when the 9-hydroxy group is α then:

(a) $R^{2'}$-$R^{3'}$ is not n-pentyl; and

(b) when $R^{1'}$ is methyl and the 13 and 14 positions are singly bonded or when $R^{1'}$ is ethyl and the 13 and 14 positions are doubly bonded, then $R^{2'}$-$R^{3'}$ is not 1,1-dimethylpentyl; and

(ii) when the 9-hydroxy group is β,

(a) when $R^{1'}$ is ethyl and the 13 and 14 positions are doubly bonded then $R^{2'}$-$R^{3'}$ is not n-pentyl or 1,1-dimethylpentyl; and

(b) when $R^{2'}$-$R^{3'}$ is n-pentyl and the 13 and 14 positions are singly bonded then $R^{1'}$ is not $C_{1-6}$ alkyl;

or a cyclodextrin clathrate thereof.

**2.** A prostaglandin F ester according to claim 1 wherein $R^{1'}$ is cyclohexyl, phenyl, benzyl or isopropyl; and $R^{2'}$-$R^{3'}$ is n-pentyl or 1,1-dimethylpentyl.

**3.** A prostaglandin F ester according to claim 2 which is:

13,14-dihydro-PGF2β cyclohexyl ester;

13,14-dihydro-PGF2β phenyl ester;

13,14-dihydro-PGF2β benzyl ester;

16,16-dimethyl-13,14-dihydro-PGF2α isopropyl ester;

16,16-dimethyl-PGF2α isopropyl ester; or

16,16-dimethyl-PGF2β isopropyl ester.

**4.** A process for the preparation of a prostaglandin F ester of formula (Ia) :

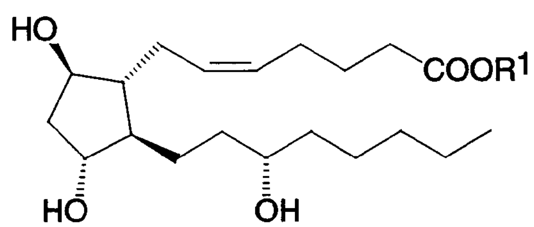

( Ia )

wherein $R^1$ is a $C_{4-7}$ carbocycle or a $C_{1-4}$ alkyl which is substituted by a $C_{4-7}$ carbocycle; the carbocycle being optionally substituted by one or more groups independently selected from $C_{1-4}$ alkyl, $C_{1-4}$ alkoxy, halogen, nitro or trifluoromethyl, which comprises hydrolyzing a compound of formula (II) :

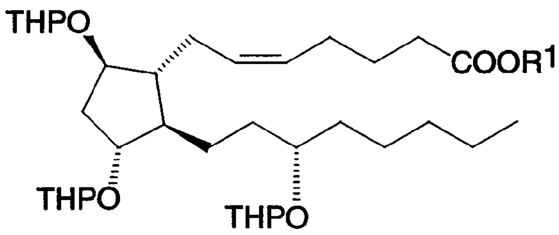

( II )

wherein THP is tetrahydropyran-2-yl and $R^1$ is as defined above, under acid conditions.

**5.** A process for the preparation of a prostaglandin F ester of formula (VIa) :

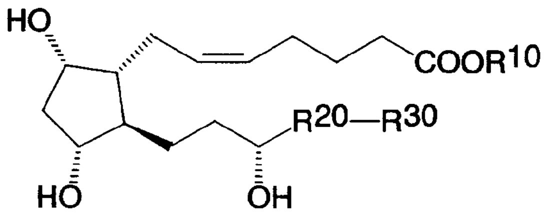

( VIa )

wherein $R^{10}$ is $C_{1-6}$ alkyl, $C_{4-7}$ carbocycle or $C_{1-4}$ alkyl which is substituted by a $C_{4-7}$ carbocycle; $R^{20}$ is a bond or $C_{1-4}$ alkylene; $R^{30}$ is $C_{1-7}$ alkyl; the carbocycle being optionally substituted by one or more groups independently selected from $C_{1-4}$ alkyl, $C_{1-4}$ alkoxy, halogen, nitro or trifluoromethyl; with the provisos that: $R^{20}$-$R^{30}$ is not n-pentyl; and when $R^{10}$ is methyl then $R^{20}$-$R^{30}$ is not 1,1-dimethylpentyl; which comprises hydrolyzing a compound of formula (XII) :

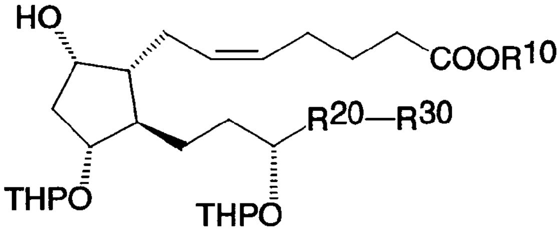

( XII )

wherein THP is tetrahydropyran-2-yl and $R^{10}$, $R^{20}$ and $R^{30}$ are as defined above, under acid conditions.

**6.** A process for the preparation of a prostaglandin F ester of formula (VIb) :

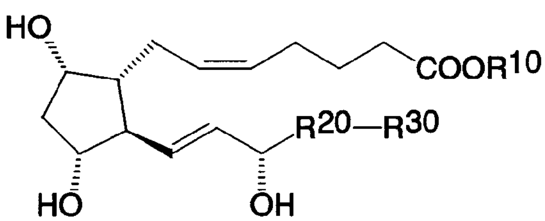

( VIb )

wherein $R^{10}$ is $C_{1-6}$ alkyl, $C_{4-7}$ carbocycle or $C_{1-4}$ alkyl which is substituted by a $C_{4-7}$ carbocycle; $R^{20}$ is a bond or $C_{1-4}$ alkylene; $R^{30}$ is $C_{1-7}$ alkyl; the $C_{4-7}$ carbocycle being optionally substituted by one or more groups independently selected from $C_{1-4}$ alkyl, $C_{1-4}$ alkoxy, halogen, nitro or trifluoromethyl; with the pro-

visos that: $R^{20}$-$R^{30}$ is not n-pentyl; and when $R^{10}$ is ethyl then $R^{20}$-$R^{30}$ is not 1,1-dimethylpentyl; which comprises esterifying a compound of formula (XIII) :

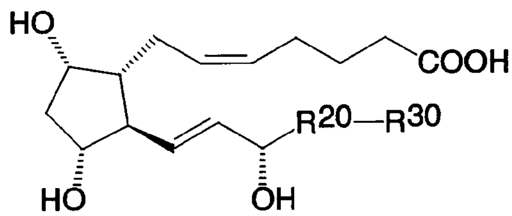

( XIII )

wherein $R^{20}$ and $R^{30}$ are as defined above.

7. A process for the preparation of a prostaglandin F ester of formula (XIV) :

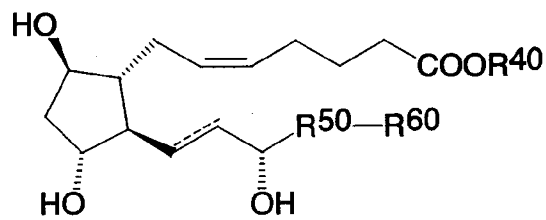

( XIV )

wherein $R^{40}$ is $C_{1-6}$ alkyl, $C_{4-7}$ carbocycle or $C_{1-4}$ alkyl which is substituted by a $C_{4-7}$ carbocycle; $R^{50}$ is a bond or $C_{1-4}$ alkylene; $R^{60}$ is $C_{1-7}$ alkyl; the $C_{4-7}$ carbocycle being optionally substituted by one or more groups independently selected from $C_{1-4}$ alkyl, $C_{1-4}$ alkoxy, halogen, nitro or trifluoromethyl; with the provisos that: $R^{50}$-$R^{60}$ is not n-pentyl; and when $R^{40}$ is ethyl and the 13- and 14- positions are doubly bonded then $R^{50}$-$R^{60}$ is not n-pentyl or 1,1-dimethylpentyl;
which comprises hydrolyzing a compound of formula (XIX) :

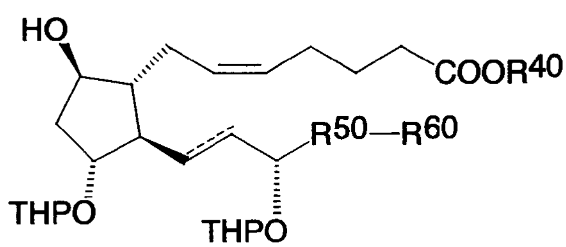

( XIX )

wherein THP is tetrahydropyran-2-yl and $R^{40}$, $R^{50}$ and $R^{60}$ are as defined above; under acid conditions.

8. A pharmaceutical composition which comprises, as active ingredient, an effective amount of a prostaglandin F ester as defined in claim 1 or a cyclodextrin clathrate thereof, with a pharmaceutical carrier or coating.

9. A prostaglandin F ester of formula (Ib) :

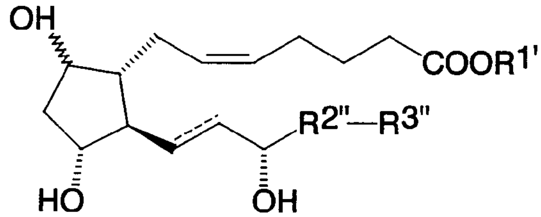

(I b)

wherein $R^{1''}$ is a $C_{1-6}$ alkyl, a $C_{4-7}$ carbocycle or a $C_{1-4}$ alkyl substituted by a $C_{4-7}$ carbocycle, the $C_{4-7}$ carbocycle being optionally substituted by one or more groups independently selected from $C_{1-4}$ alkyl, $C_{1-4}$ alkoxy, halogen, nitro and trifluoromethyl :

$R^{2''}$ is a bond or $C_{1-4}$ alkylene;
$R^{3''}$ is $C_{1-7}$ alkyl; and
the 9-hydroxy group is $\alpha$ or $\beta$;
with the provisos that:

(i) when the 9-hydroxy group is $\alpha$ then:
(a) $R^{2''}$-$R^{3''}$ is not n-pentyl; and
(b) when $R^{1''}$ is methyl and the 13 and 14 positions are singly bonded or when $R^{1''}$ is ethyl and the 13 and 14 positions are doubly bonded, then $R^{2''}$-$R^{3''}$ is not 1,1-dimethylpentyl; and

(ii) when the 9-hydroxy group is $\beta$,
(a) when $R^{1''}$ is ethyl and the 13 and 14 positions are doubly bonded then $R^{2''}$-$R^{3''}$ is not n-pentyl; and
(b) when $R^{2''}$-$R^{3''}$ is n-pentyl and the 13 and 14 positions are singly bonded then $R^{1''}$ is not $C_{1-6}$ alkyl;
or a cyclodextrin clathrate thereof; for use in a method of treatment of the human or animal body.

**10.** Use of a prostaglandin F ester or a cyclodextrin clathrate thereof as defined in claim 9 in the manufacture of a medicament for use in the treatment of glaucoma.

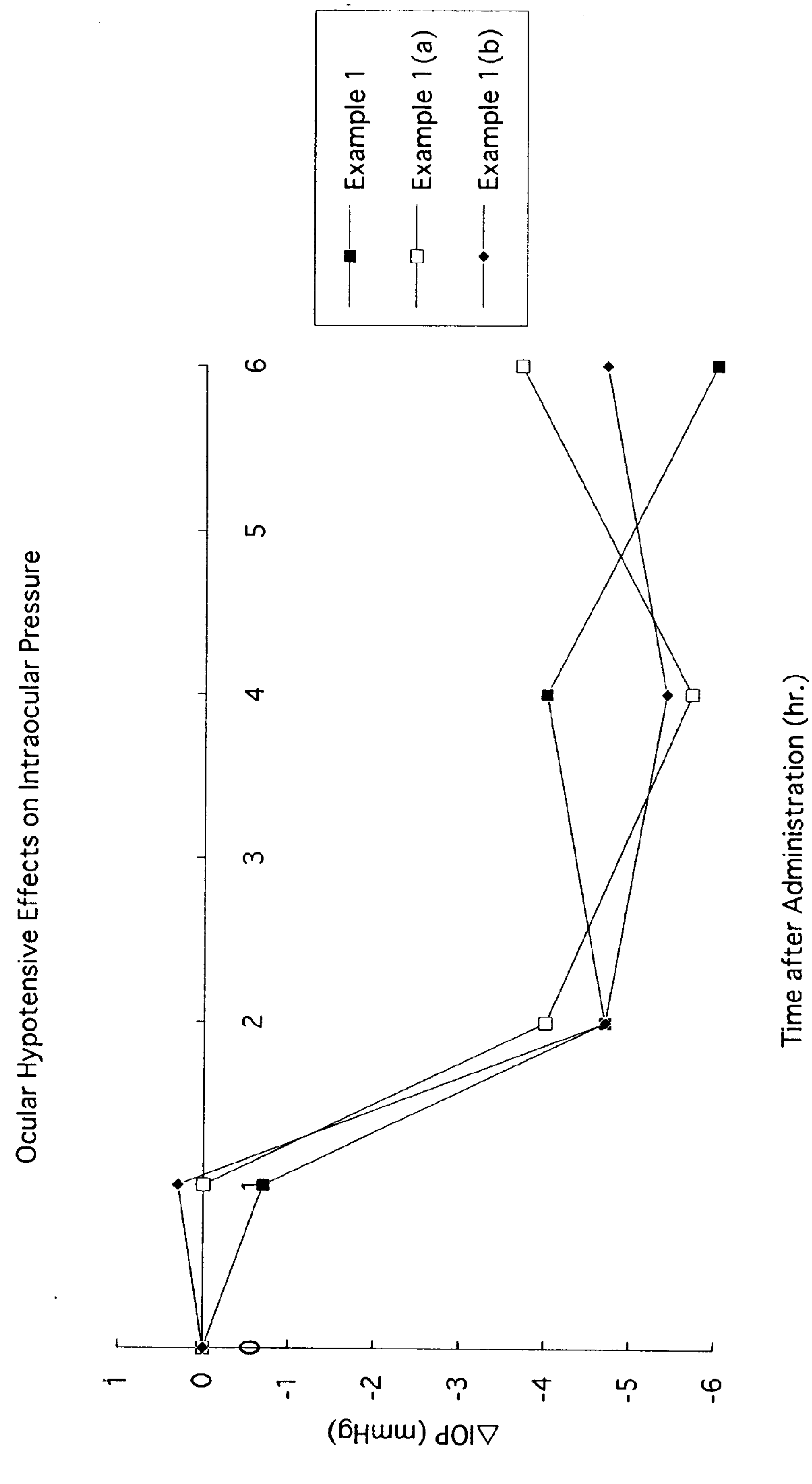
Ocular Hypotensive Effects on Intraocular Pressure
ΔIOP (mmHg)
1
0
-1
-2
-3
-4
-5
-6
0
1
2
3
4
5
6
Time after Administration (hr.)
Example 1
Example 1 (a)
Example 1 (b)

Fig. 1

Fig. 2

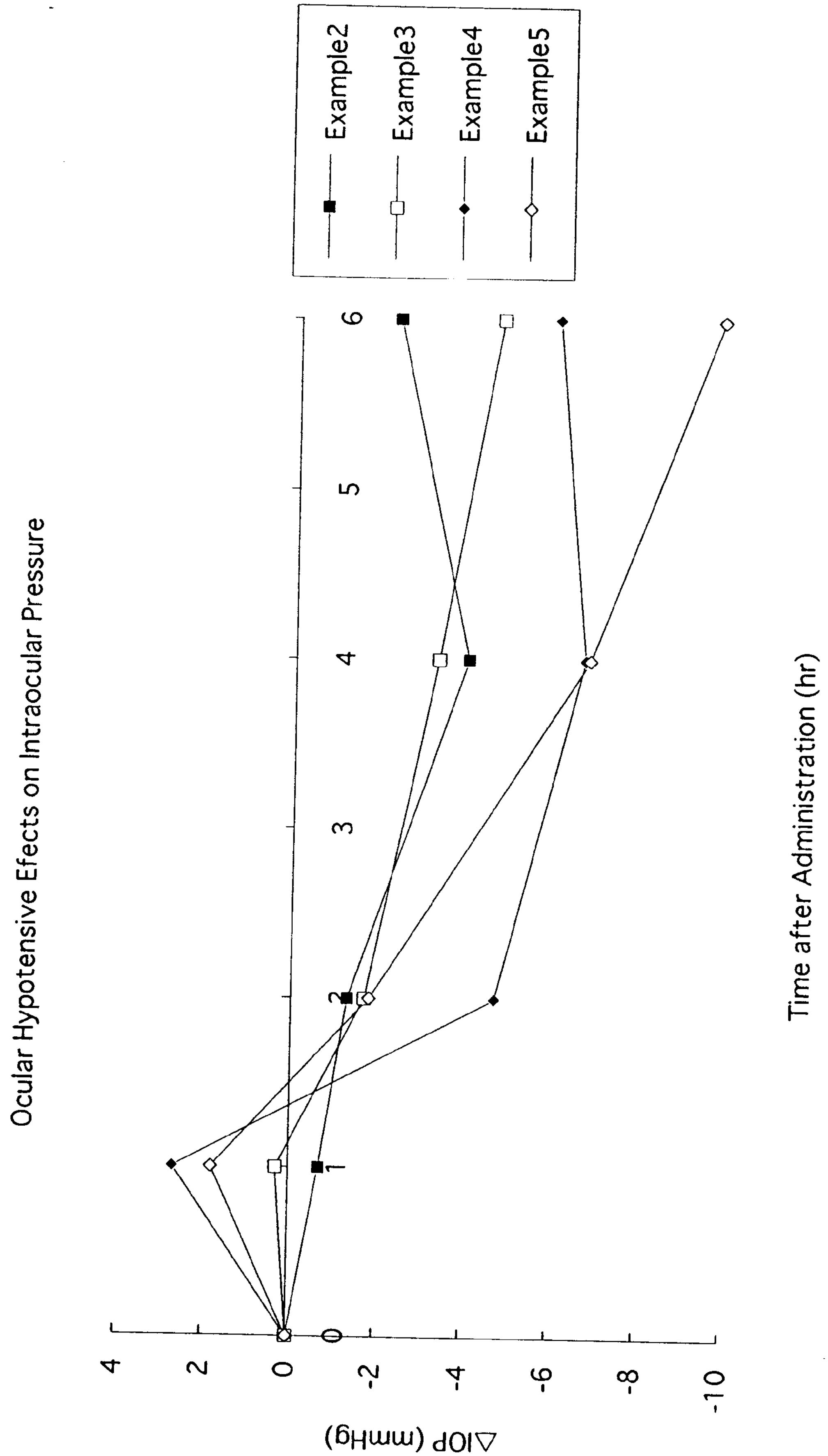

Ocular Hypotensive Efects on Intraocular Pressure
ΔIOP (mmHg)
4
2
0
-2
-4
-6
-8
-10
0
1
2
3
4
5
6
Time after Administration (hr)
Example2
Example3
Example4
Example5

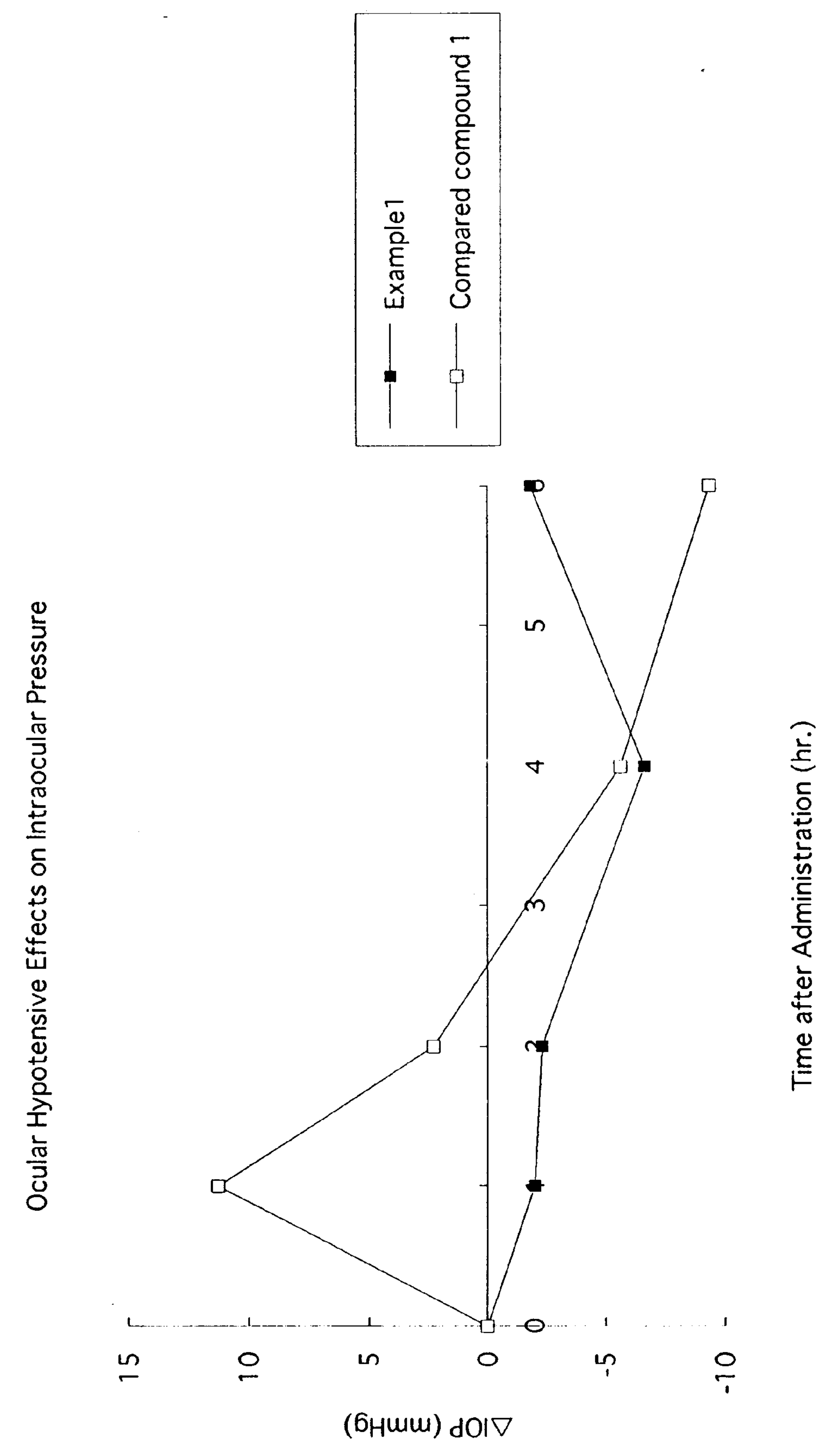
Ocular Hypotensive Effects on Intraocular Pressure
ΔIOP (mmHg)
15
10
5
0
-5
-10
0
2
3
4
5
6
Time after Administration (hr.)
Example1
Compared compound 1

Fig. 3

Fig. 4

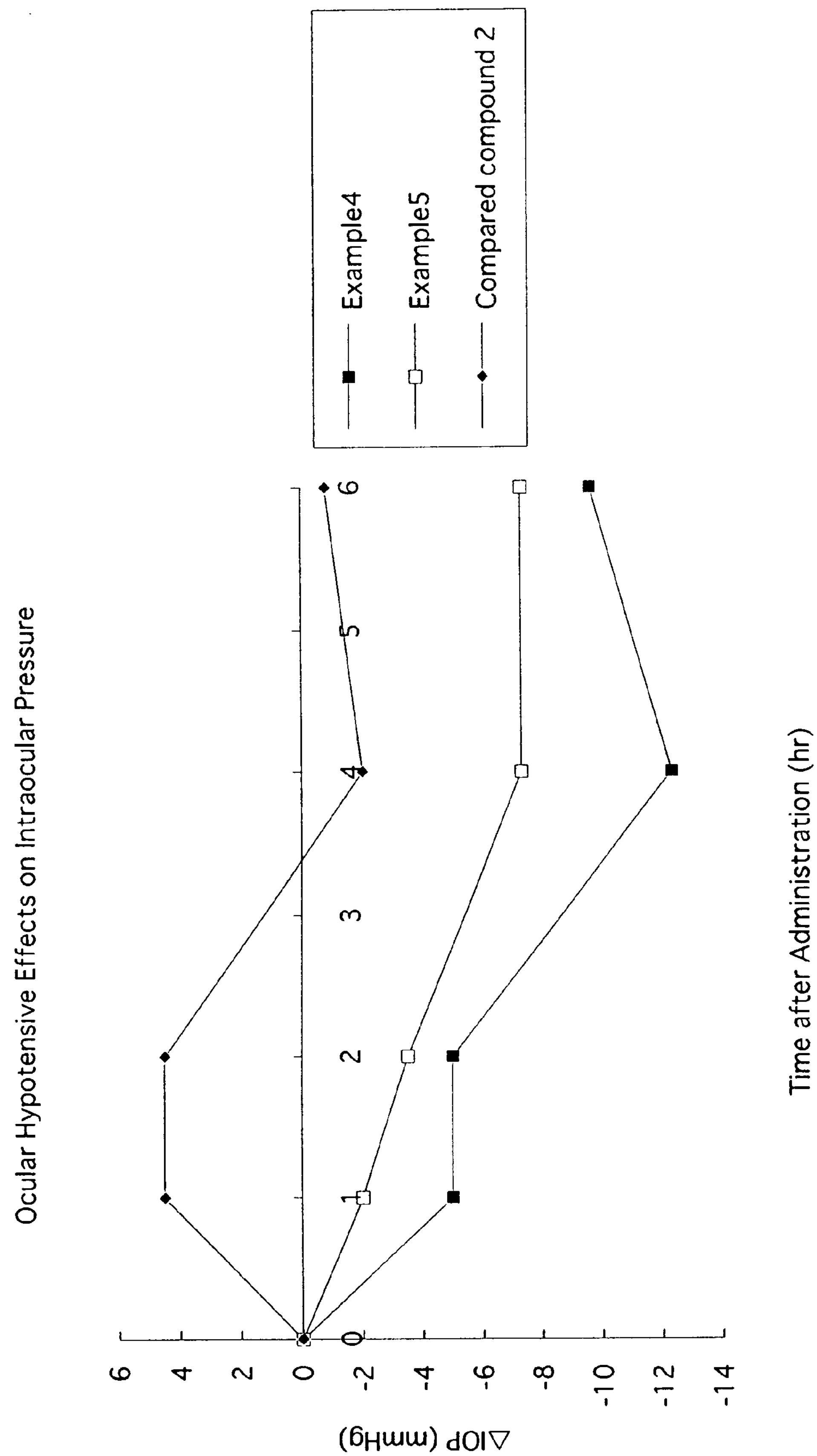
Ocular Hypotensive Effects on Intraocular Pressure
Example4
Example5
Compared compound 2
ΔIOP (mmHg)
6
4
2
0
-2
-4
-6
-8
-10
-12
-14
0
1
2
3
4
5
6
Time after Administration (hr)